# EUROPEAN PATENT APPLICATION

(11) **EP 2 314 278 A1**
(43) Date of publication of application: **27.04.2011**
(21) Application number: 09806564.2
(22) Date of filing: 06.08.2009
(51) Int. Cl.: A61K 8/64, A61K 8/36, A61Q 5/00, A61Q 5/12, C11D 3/20, C11D 3/33, C11D 3/34

(54) **HAIR TREATMENT COMPOSITION**

(30) Priority: 12.08.2008 JP 2008207985; 24.06.2009 JP 2009150150
(71) Applicant: Kao Corporation, Chuo-Ku Tokyo 103-8210 (JP)
(72) Inventor: NOJIRI, Masayoshi, Tokyo 131-8501 (JP); SAZANAMI, Fumiko, Tokyo 131-8501 (JP)
(74) Representative: HOFFMANN EITLE
(86) International application number: PCT/JP2009/003766
(87) International publication number: WO 2010/018668

(57) **Abstract**

The present invention is a hair treatment composition containing components (a) and (b): (a) a compound having 2 or 3 amino acid residues and represented by general Formula (1), or a salt thereof [in the formula, X denotes a divalent hydrocarbon group having 1 to 4 carbon atoms, which may be substituted with a hydroxy group, or a specific amino acid residue, Y denotes a specific amino acid residue or a divalent group represented by chemical Formula (2) (in the formula, -* denotes a direct bond bonding to an adjacent carbonyl group or oxygen atom), R denotes a hydrogen atom or a monovalent hydrocarbon group having 1 to 4 carbon atoms, which may be substituted with a hydroxy group, m and n denote 0 or 1, and when m and n are simultaneously 1, X is not an amino acid residue]; and (b) an aliphatic carboxylic acid having no greater than 8 carbon atoms or a salt thereof.

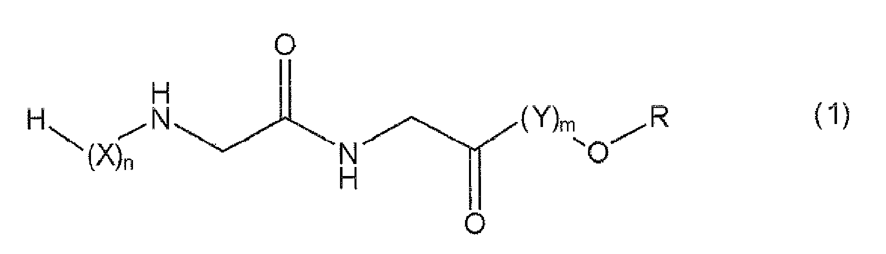

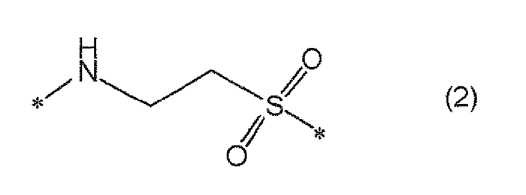

## Description

### FIELD OF THE INVENTION

The present invention relates to a hair treatment composition.

### BACKGROUND OF THE INVENTION

'Morning hair' is the phenomenon of head hair being formed into an undesirable shape by external forces applied to the hair from bedding and so on during sleep, and it is an inconvenient phenomenon that many people experience in daily life.
When correcting morning hair, once hair was humidified to break hydrogen bonds present within the hair, then reforming the hydrogen bonds in a more preferred shape while drying with a dryer and so on, and fixing between hairs by applying various setting polymers to the hair surface in order to maintain the shape are carried out. Such operations are complicated, and unless fixation between hairs by a setting polymer is carried out it is not satisfactory in terms of sustaining styling.

On this point, as a technique for preventing the occurrence of morning hair, Japanese Patent Application Laid-open No. 8-198725 proposes a cosmetic for head hair formed by formulating a protein hydrolysate called conchiolin and so on with trimethylglycine. However, this technique is a technique for enhancing moisture retention within hair so as to promote dissociation of hydrogen bonds within the hair and preventing hair from drying during sleep and being fixed in an undesirable shape, and hydrogen bond exchange within the hair continues even after finishing styling. Because of this, there is instead the problem that it is difficult to maintain the finished styling.

In order to easily return head hair with morning hair to its original shape, that is, to easily carry out styling, some techniques for reforming the internal hair structure have been proposed. In all of these techniques, hydrogen bonds present within the hair are broken so as to make styling easy, and imparting and repairing a shape become easy. However, it can't be avoided the problem that undesirable dissociation/reformation of hydrogen bonds are continuing to proceed even after finishing styling, which makes it difficult to keep the finished style. For example, patent document 2 proposes a hair treatment composition to give excellent luster and smoothness that contains at least two or more kinds of α-hydroxycarboxylic acid, a protein decomposition product, and a highly polymerized dimethylpolysiloxane. Furthermore, patent document 3-proposes a hair treatment composition that contains an α-hydroxycarboxylic acid or a salt thereof and a specific plant extract and that provides luster and softness. However, none of these technical proposals can satisfactorily sustain styling.

### Prior art document

### Patent documents

Patent document 1: Japanese Patent Application Laid-open No. 8-198725
Patent document 2: Japanese Patent Application Laid-open No. 10-81614
Patent document 3: Japanese Patent Application Laid-open No. 11-5719

### Summary of the invention

As described above, sustaining styling while preventing morning hair or making styling easy is mutually contradictory demands, and there is no conventional technique that can simultaneously achieve the above.

It is an object of the present invention to provide a hair treatment composition that can simultaneously impart softness, luster, and alignment and can prevent morning hair, make styling easy, and also sustain the styling.

The present inventors have found that the use of a hair composition that contains a glycylglycine (a) as a moisturizing agent that retains water within hair, which is a substance that acts to widen the hydrogen bond network, and a specific aliphatic carboxylic acid (b) that acts to disturb the hydrogen bond network enables the mutually contradictory demands to be simultaneously satisfied, and it is possible to make the styling of hair easy while preventing the occurrence of morning hair and, moreover, to maintain the styling.
(a) A compound having 2 or 3 amino acid residues and represented by general Formula (1) or a salt thereof:

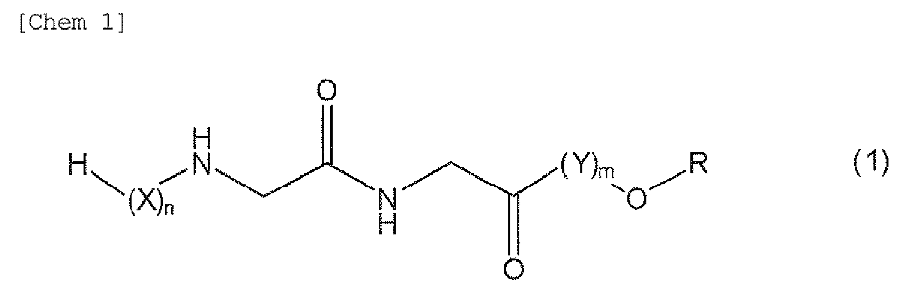

[In the formula, X denotes a divalent hydrocarbon group having 1 to 4 carbon atoms, which may be substituted with a hydroxy group, or an amino acid residue selected from an arginine residue, an alanine residue, a phenylalanine residue, a glycine residue, a glutamine residue, a glutamic acid residue, a serine residue, a proline residue, an N-methylproline residue, and a 4-hydroxyproline residue.

Y denotes an amino acid residue selected from an arginine residue, an alanine residue, a glycine residue, a glutamine residue, a glutamic acid residue, a serine residue, a proline residue, and a 4-hydroxyproline residue, or a divalent group represented by chemical Formula (2).

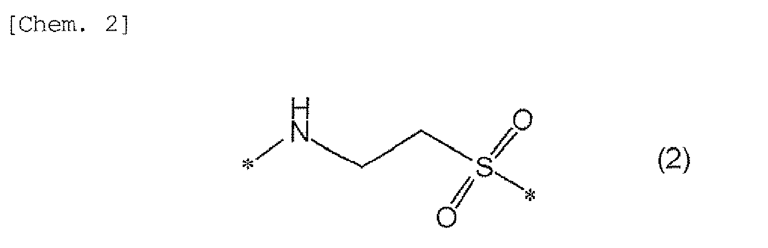

(In the formula, -* denotes a direct bond bonding to an adjacent carbonyl group or oxygen atom.)

R denotes a hydrogen atom or a monovalent hydrocarbon group having 1 to 4 carbon atoms, which may be substituted with a hydroxy group. m and n denote 0 or 1. When m and n are simultaneously 1, X is not an amino acid residue.]; (b) an aliphatic carboxylic acid having no greater than 8 carbon atoms or a salt thereof.

In accordance with the present invention, there can be provided a hair treatment composition that simultaneously imparts softness, luster, and alignment to dry hair after treatment, prevents morning hair, makes styling easy and, moreover, sustains the styling.

### BRIEF DESCRIPTION OF THE DRAWINGS

FIG. 1 schematically shows a bending angle θ of a hair bundle for evaluating susceptibility to morning hair, and
FIG. 2 schematically shows a curl diameter of a hair bundle for evaluating ease of styling and sustainability of styling.

### DETAILED DESCRIPTION OF THE INVENTION

The present invention is explained below.
The hair treatment composition of the present invention contains component (a) and component (b) below.
(a) A compound having 2 or 3 amino acid residues and represented by general Formula (1) or a salt thereof; and.
(b) an aliphatic carboxylic acid having no greater than 8 carbon atoms or a salt thereof.

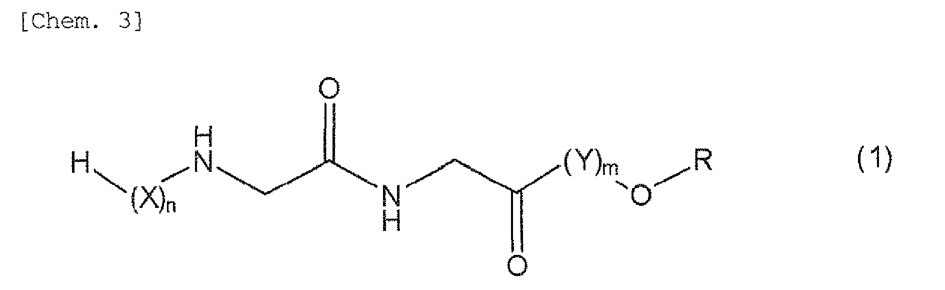

Component (a) is a compound represented by general Formula (1) or a salt thereof; it may be a free form, or may be a salt or an intramolecular salt (amphoteric iron).
Examples of the salt of compound (a) include inorganic acid salts such as a hydrochloride and a sulfate; ammonium salts such as ammonium and an alkylammonium salt; alkali metal salts such as a sodium salt and a potassium salt; and alkaline earth metal salts such as a calcium salt and a magnesium salt.

In general Formula (1), X denotes a divalent hydrocarbon group having 1 to 4 carbon atoms, which may be substituted with a hydroxy group, or an amino acid residue selected from an arginine residue, an alanine residue, a phenylalanine residue, a glycine residue, a glutamine residue, a glutamic acid residue, a serine residue, a proline residue, an N-methylproline residue, and a 4-hydroxyproline residue. n is 0 or 1.
The divalent hydrocarbon group having 1 to 4 carbon atoms, which may be substituted with a hydroxy group, may be a saturated or unsaturated, straight-chain or branched group. Among them, a divalent saturated hydrocarbon group or a divalent saturated hydrocarbon group that is substituted with a hydroxy group is preferable.

Examples of the divalent saturated hydrocarbon group include a methylene group, an ethylene group, an ethylidene group, a vinylene group, a trimethylene group, an isopropylidene group, a 1-propenylene group, a tetramethylene group, a 2-methyltrimethylene group, a 1-methyltrimethylene group, and a 1-butenylene group.
Examples of the divalent saturated hydrocarbon group that is substituted with a hydroxy group include a 1-hydroxyethylene group, a 1-hydroxytrimethylene group, a 1,2-dihydroxytrimethylene group, a 1-hydroxytetramethylene group, a 1,2-dihydroxytetramethylene group, a 1,3-dihydroxytetramethylene group, and a 1,2,3-trihydroxytetramethylene group.

In general Formula (1), Y denotes an amino acid residue selected from an arginine residue, an alanine residue, a glycine residue, a glutamine residue, a glutamic acid residue, a serine residue, a proline residue, and a 4-hydroxyproline residue, or a divalent group represented by chemical Formula (2) below. m is 0 or 1.

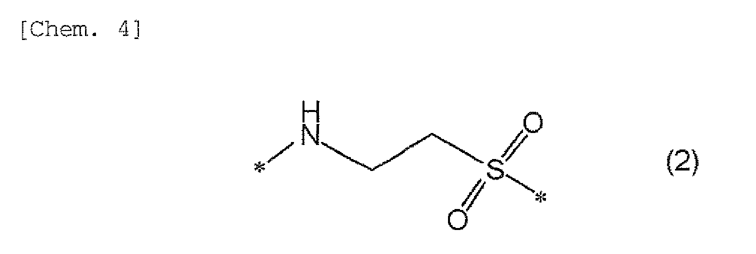

(In the formula, -* denotes a direct bond bonded to an adjacent carbonyl group or oxygen atom.)

Furthermore, in general Formula (1), m and n denote 0 or 1. When m and n are simultaneously 1, X is not an amino acid residue. That is, when m = n = 1, X is a divalent hydrocarbon group having 1 to 4 carbon atoms, which may be substituted with a hydroxy group.

In general Formula (1), R denotes a hydrogen atom or a monovalent hydrocarbon group having 1 to 4 carbon atoms, which may be substituted with a hydroxy group.
The monovalent hydrocarbon group having 1 to 4 carbon atoms, which may be substituted with a hydroxy group, may be a saturated or unsaturated, straight-chain or branched group.
The monovalent hydrocarbon group having 1 to 4 carbon atoms is preferably an alkyl group, and examples thereof include a methyl group, an ethyl group, a propyl group, a butyl group, an isopropyl group, an isobutyl group, a sec-butyl group, and a tert-butyl group.
The monovalent hydrocarbon group having 1 to 4 carbon atoms that is substituted with a hydroxy group is preferably a hydroxyalkyl group, and examples thereof include a 2-hydroxyethyl group, a 3-hydroxypropyl group, a 4-hydroxybutyl group, a 2,3-dihydroxypropyl group, a 2,3,4-trihydroxybutyl group, and a 2,4-dihydroxybutyl group.

Compound examples suitable for component (a) include compounds represented by Formulae (G1) to (G9) below. Among them, (G2) to (G9) are more preferable, and glycylglycylglycine (G8) and glycylglycine (G9) are yet preferable. These compounds may be in a free form, may be an amphoteric ion, or may form a salt. Furthermore, they may be used singly or in a combination of at least two or more kinds.

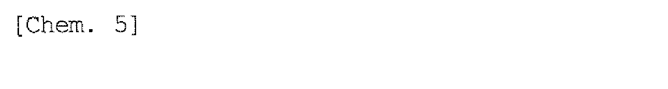

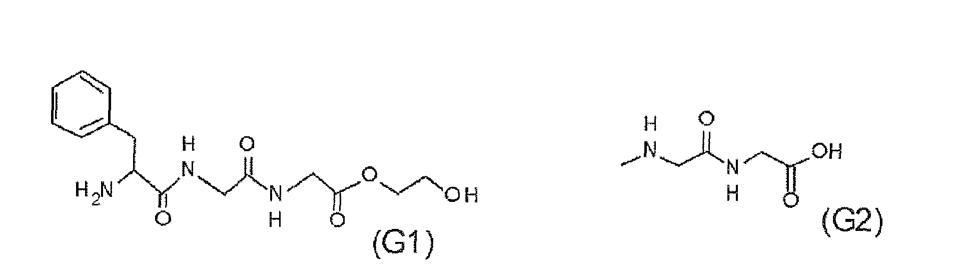

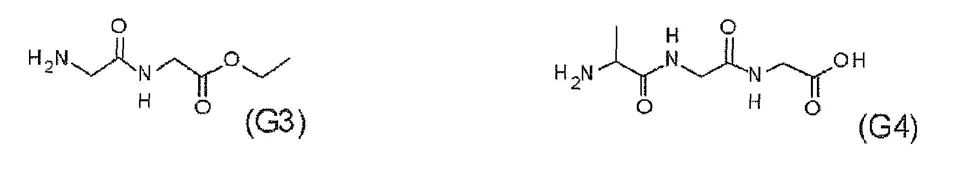

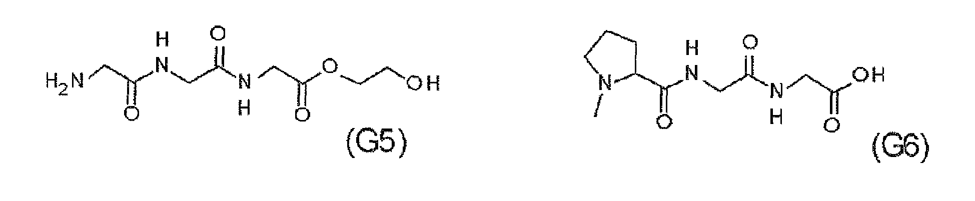

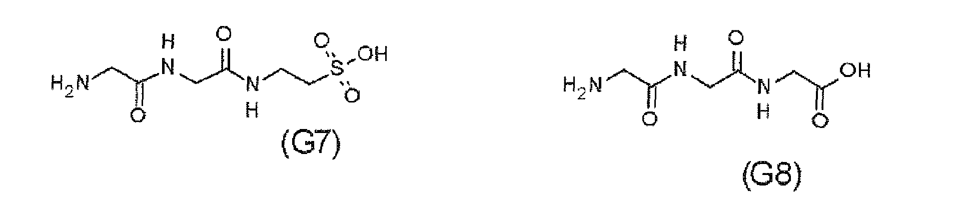

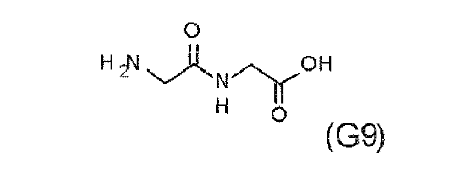

The content of component (a) in the composition is preferably 0.01 to 10 mass %, more preferably 0.03 to 5 mass %, and yet more preferably 0.05 to 3 mass %, from the viewpoint of preventing the composition from turning yellow.

Component (b) is an aliphatic carboxylic acid having no greater than 8 carbon atoms or a salt thereof. Examples of the salt of compound (b) include ammonium salts such as ammonium salt and an alkylammonium salt; alkali metal salts such as a sodium salt and a potassium salt; and alkaline earth metal salts such as a calcium salt and a magnesium salt. Among them, an ammonium salt, a sodium salt, and a potassium salt are preferable. Preferred examples of component (b) include malic acid, citric acid, lactic acid, glycolic acid, pyrrolidone carboxylic acid, malonic acid, maleic acid, nicotinic acid, benzoic acid, glyceric acid, and tartaric acid, and salts thereof. Malic acid, citric acid, lactic acid, glycolic acid, glyceric acid, or tartaric acid, or a salt thereof is preferable.
Furthermore, component (b) may be formulated as a counterion of component (a).
The content of component (b) in the composition is preferably 0.01 to 10 mass %, more preferably 0.03 to 5 mass %, and yet more preferably 0.05 to 3 mass %, from the viewpoint of preventing the composition from turning yellow. Furthermore, they may be used singly or in a combination of at least two or more kinds.

The hair treatment composition of the present invention may contain as component (b') an aromatic sulfonic acid having no greater than 10 carbon atoms or a salt thereof. Examples of the salt of compound (b') include ammonium salts such as ammonium and an alkylammonium salt; alkali metal salts such as a sodium salt and a potassium salt; and alkaline earth metal salts such as a calcium salt and a magnesium salt. Among them, an ammonium salt, a sodium salt, and a potassium salt are preferable. Preferred examples of component (b') include benzenesulfonic acid, p-toluenesulfonic acid, 2,4-dimethylbenzenesulfonic acid, 2,5-dimethylbenzenesulfonic acid, naphthalenesulfonic acid, and saccharin, and salts thereof. Among them, p-toluenesulfonic acid or naphthalenesulfonic acid, or a salt thereof is preferable.
The content of component (b') in the composition is preferably 0.01 to 10 mass %, more preferably 0.03 to 5 mass %, and yet more preferably 0.05 to 3 mass %, from the viewpoint of preventing the composition from turning yellow. Furthermore, they may be used singly or in a combination of at least two or more kinds.
The hair treatment composition of the present invention may further contain an organic solvent as component (c). Examples of the organic solvent include lower alkanols such as ethanol and 2-propanol; aromatic alcohols such as benzyl alcohol and 2-benzyloxyethanol; polyols such as propylene glycol, 1,3-butanediol, diethylene glycol, and glycerol; CELLOSOLVES such as ethyl CELLOSOLVE and butyl CELLOSOLVE; and carbitols such as ethylcarbitol and butylcarbitol.

Furthermore, the hair treatment composition of the present invention may further contain a surfactant as component (d). As such a surfactant, any of a cationic surfactant, a nonionic surfactant, an amphoteric surfactant, and an anionic surfactant may be used. Among them, a cationic surfactant or an anionic surfactant is suitably used.

Examples of the cationic surfactant include a mono-long-chain-alkyl quaternary ammonium salt, a di-long-chain-alkyl quaternary ammonium salt, a long-chain-alkoxyalkyl quaternary ammonium salt, a long-chain-alkylamide quaternary ammonium salt, a long-chain-alkyl tertiary amine and a salt thereof, a long-chain-alkoxyalkyl tertiary amine and a salt thereof, and a long-chain-alkylamide tertiary amine and a salt thereof.
Among them, examples of the long-chain-alkoxyalkyl tertiary amine and a salt thereof include a long-chain-alkyl ether tertiary amine represented by general Formula (F1) and a salt thereof, and a hydroxy long-chain-alkyl ether tertiary amine represented by general Formula (F2) and a salt thereof.

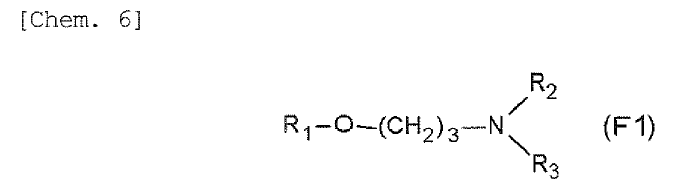

In the formula, R1 denotes a straight-chain or branched alkyl or alkenyl group having 6 to 24 carbon atoms. R2 and R3 independently denote an alkyl group having 1 to 6 carbon atoms or an -(AO)mH group. In the -(AO)mH group, A denotes an alkylene group having 2 to 4 carbon atoms, m denotes a number from 1 to 6, the m AOs may be identical to or different from each other, and they may have any arrangement.

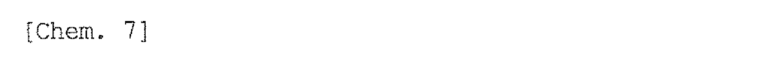

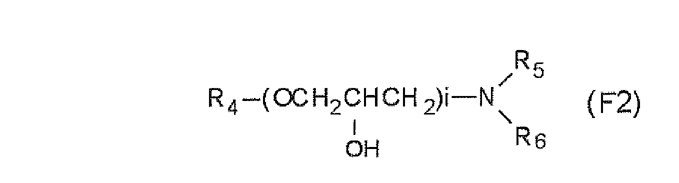

In the formula, R4 denotes a straight-chain or branched alkyl or alkenyl group having 6 to 24 carbon atoms. R5 and R6 independently denote an alkyl group having 1 to 6 carbon atoms or an -(AlO)bH group. In the -(AlO)bH group, A1 denotes an alkylene group having 2 to 4 carbon atoms, b denotes a number from 1 to 6, the b AlOs may be identical to or different from each other, and they may have any arrangement. i denotes a number from 1 to 5.

Specific examples thereof include cetyltrimethylammonium chloride, stearyltrimethylammonium chloride, behenyltrimethylammonium chloride, stearyldimethylbenzylammonium chloride, a benzalkonium chloride, distearyldimethylammonium chloride, N,N-dimethyl-3-hexadecyloxypropylamine and a salt thereof, and N,N-dimethyl-3-octadecyloxypropylamine and a salt thereof. Among them, stearyltrimethylammonium chloride, behenyltrimethylammonium chloride, distearyldimethylammonium chloride, and N,N-dimethyl-3-octadecyloxypropylamine and a salt thereof are preferable.

Examples of the anionic surfactant include an alkylbenzenesulfonic acid salt, an alkyl or alkenyl ether sulfate, an alkyl or alkenyl sulfate, an olefinsulfonic acid salt, an alkanesulfonic acid salt, a saturated or unsaturated fatty acid salt, an alkyl or alkenyl ether carboxylic acid salt, an α-sulfofatty acid salt, an N-acylamino acid salt, a phosphoric acid mono or diester salt, and a sulfosuccinic acid ester salt. Among them, an alkyl ether sulfate is preferable, and specific examples thereof include a polyoxyethylene alkyl ether sulfate. Examples of the counterion of the anionic group of these anionic surfactants include alkali metal ions such as sodium ion and potassium ion; alkaline earth metal ions such as calcium ion and magnesium ion; ammonium ion; and an alkanolamine having 1 to 3 alkanol groups having 2 or 3 carbon atoms (for example, monoethanolamine, diethanolamine, triethanolamine, or triisopropanolamine).

Examples of the nonionic surfactant include a polyoxyalkylene alkyl ether, a polyoxyalkylene alkenyl ether, a higher fatty acid sucrose ester, a polyglycerol fatty acid ester, a higher fatty acid mono- or di-ethanolamide, a polyoxyethylene hardened castor oil, a polyoxyethylene sorbitan fatty acid ester, a polyoxyethylene sorbit fatty acid ester, an alkyl saccharide, an alkylamine oxide, an alkylamidoamine oxide, an alkyl glyceryl ether, and an alkenyl glyceryl ether. Among them, a polyoxyalkylene alkyl ether, a higher fatty acid mono- or di-ethanolamide, a polyoxyethylene hardened castor oil, and an alkyl glyceryl ether are preferable, and a polyoxyethylene alkyl (average number of carbon atoms 12 to 14) ether, 2-ethylhexyl glyceryl ether, and isodecyl glyceryl ether are more preferable.

Examples of the amphoteric surfactant include an imidazoline type, a carbobetaine, an amidobetaine, a sulfobetaine, a hydroxysulfobetaine, and an amidosulfobetaine.

The content of the surfactant depends on the intended application of the hair treatment agent and the function (improvement of feel, emulsifying ability, cleaning ability and so on) of each surfactant. For example, it is preferably 0.01 to 20 mass % in the composition, more preferably 0.1 to 15 mass %, and yet more preferably 0.5 to 10 mass %.

Furthermore, the hair treatment composition of the present invention may further contain a conditioning component as component (e). The conditioning component referred to here is selected from a cationic polymer, a silicones, and an oil agent.

The cationic polymer is used for the purpose of improving the feel of hair during use. The cationic polymer referred to here means a polymer having a cationic group or a group that can be ionized to be cationic, and includes an amphoteric polymer that becomes cationic in its entirety. That is, examples of the cationic polymer include water-soluble polymers containing an amino group or an ammonium group in a side chain of the polymer chain, or a diallyl quaternary ammonium salt as a constituent unit. Specific examples thereof include cationized cellulose, cationized starch, cationized guar gum, cationized tara gum, cationized fenugreek gum, cationized locust bean gum, a polymer or copolymer of a diallyl quaternary ammonium salt, and a quaternized polyvinylpyrrolidone. Among them, from the viewpoint of softness, smoothness, and ease of running fingers through during rinsing and during drying, the effects of manageability and moisture retention during drying, and agent stability, a polymer containing a diallyl quaternary ammonium salt as a constituent unit, a quaternized polyvinylpyrrolidone, and a cationized cellulose are preferable, and a polymer or copolymer of a diallyl quaternary ammonium salt and a cationized cellulose are more preferable.

Specific examples of the cationic polymer include a dimethyldiallylammonium chloride polymer (polyquaternium-6, for example, MERQUAT 100; Nalco Company), a dimethyldiallylammonium chloride/acrylic acid copolymer (polyquaternium-22, for example, MERQUAT 280 and 295; Nalco Company), a dimethyldiallylammonium chloride/acrylamide copolymer (polyquaternium-7, for example, MERQUAT 550; Nalco Company), a quaternized polyvinylpyrrolidone (GAFQUAT 734, 755, and 755N; ISP Japan Ltd.), a cationized cellulose (Leogard G and GP; Lion Corporation, polymer JR-125, JR-400, JR-30M, LR-400, and LR-30M; all from Union Carbide corporation), and cationized gums (Catinal CTR-100, CF-100, and CLB-100; TOHO Chemical Industry Co., Ltd.).

These cationic polymers may be used singly or in a combination of at least two or more kinds. The content of the cationic polymer is preferably 0.01 to 20 mass % in the composition, and more preferably 0.5 to 10 mass %, from the viewpoint of the effect in improving feel and stability toward separation of the composition.

Examples of the silicones include polydimethylsiloxanes, modified silicones (for example, amino-modified silicone, fluorine-modified silicone, alcohol-modified silicone, polyether-modified silicone, epoxy-modified silicone, alkyl-modified silicone or the like), a cyclic polydimethylsiloxane, an aminopolyether-modified silicone, and a highly polymerized methylpolysiloxane emulsion. Among them, a polydimethylsiloxane, a polyether-modified silicone, an amino-modified silicone, a cyclic polydimethylsiloxane, an aminopolyether-modified silicone, and a highly polymerized methylpolysiloxane emulsion are preferable.

These silicones may be commercially available, and examples thereof include BY11-026, BY22-19, FZ-3125, and SH200-1,000,000cs (Dow Corning Toray Co., Ltd.), TSF451-100MA (Momentive Performance Materials Japan LLC), KM-9716 (Shin-Etsu Chemical Co., Ltd.) [the above are polydimethylsiloxanes], TSF4440 (Momentive Performance Materials Japan LLC), KF-6005, KF-6011, and KF-353A (Shin-Etsu Chemical Co., Ltd.) [the above are polyether-modified silicones], SF8451C, SF8452C, SF8457C, DC929, DC8500, SS-3551, SM8704C, and SM8904 (Dow Corning Toray Co., Ltd.), KF-867 (Shin-Etsu Chemical Co., Ltd.), KT1989 (Momentive Performance Materials Japan LLC) [the above are amino-modified silicones], SH244 and SH245 (Dow Corning Toray Co., Ltd.) [the above are cyclic polydimethylsiloxanes], SS-3588 (Dow Corning Toray Co., Ltd.) [the above is an aminopolyether-modified silicone], and BY22-060 (Dow Corning Toray Co., Ltd.) [the above is a highly polymerized methylpolysiloxane emulsion].

The content of the silicone is preferably 0.1 to 20 mass % in the composition, and more preferably 0.5 to 15 mass %, from the viewpoint of a sufficient effect and suppression of tackiness.

Examples of the oil include hydrocarbons such as squalene, squalane, vaseline, paraffin, liquid paraffin, liquid isoparaffin, and cycloparaffin; glycerides such as castor oil, cocoa oil, mink oil, avocado oil, olive oil, and shea butter; waxes such as beeswax, spermaceti, lanolin, and carnauba wax; ester oils such as isopropyl palmitate, isopropyl myristate, octyldodecyl myristate, hexyl laurate, cetyl lactate, propylene glycol monostearate, oleyl oleate, hexadecyl 2-ethylhexanoate, isononyl isononanoate, and tridecyl isononanoate; higher fatty acids such as capric acid, lauric acid, myristic acid, palmitic acid, stearic acid, behenic acid, oleic acid, lanolin fatty acid, iso fatty acid, anteiso fatty acid, coconut oil fatty acid, 18-methyleicosanoic acid, 16-methyloctadecanoic acid, and a mixture of these fatty acids/branched fatty acids; higher alcohols such as myristyl alcohol, cetyl alcohol, oleyl alcohol, stearyl alcohol, isostearyl alcohol, behenyl alcohol, 2-octyldodecanol, and cetostearyl alcohol; and others such as isostearyl glyceryl ether and polyoxypropylene butyl ether. Among them, higher alcohols are preferable.
These oil agents may be used singly or in a combination of at least two or more kinds. The content thereof is preferably 0.1 to 20 mass % in the composition, and more preferably 0.5 to 15 mass %, from the viewpoint of the effect in improving feel and stability toward separation of the composition.

Since such a conditioning component (e) remains in the hair to a moderate extent, a good conditioning effect can be imparted.

The hair treatment composition of the present invention may contain another component that is normally used as a cosmetic raw material in addition to the above components. Examples of such an optional component include a thickener, a preservative, a chelating agent, a stabilizer, an antioxidant, a plant extract, a herbal extract, a protein hydrolysate, vitamins, a colorant such as a dye, a fragrance, a UV absorber, a pearlescent agent such as an ethylene glycol difatty acid ester, a setting polymer, and an amphiphilic amide lipid. Specific examples of the amphiphilic amide lipid include a diamide compound represented by general Formula (F3) and ceramides represented by general Formula (F4).

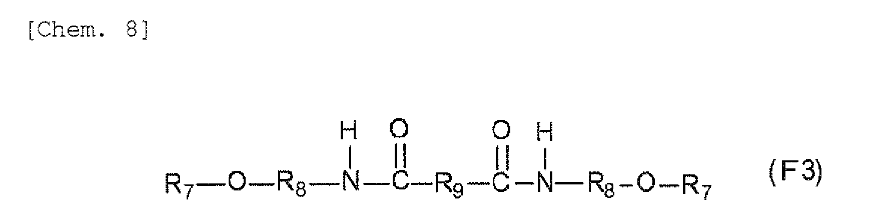

In the formula, R7 denotes a straight-chain or branched hydrocarbon group having 1 to 12 carbon atoms, which may be substituted with a hydroxy group and/or an alkoxy group. R8 denotes a straight-chain or branched divalent hydrocarbon group having 1 to 5 carbon atoms. R9 denotes a straight-chain or branched divalent hydrocarbon group having 1 to 22 carbon atoms.

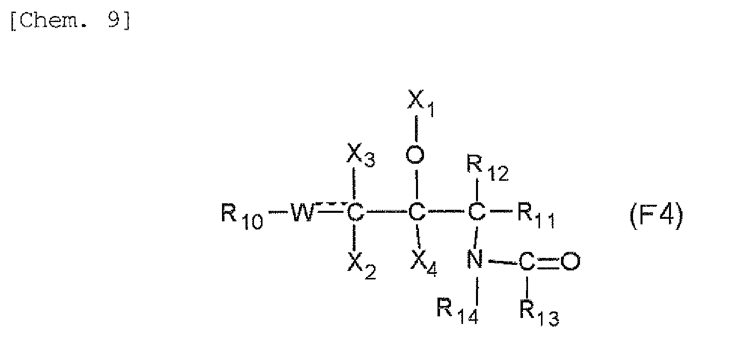

In the formula, R10 denotes a straight-chain, branched, or cyclic, saturated or unsaturated hydrocarbon group having 4 to 30 carbon atoms, which may be substituted with a hydroxy group, an oxo group, or an amino group. W denotes a methylene group, a methine group, or an oxygen atom. X1 denotes a hydrogen atom, an acetyl group, or a glyceryl group, or together with the adjacent oxygen atom forms an oxo group. X2, X3, and X4 each independently denote a hydrogen atom, a hydroxy group, or an acetoxy group. When W is a methine group, one of X2 and X3 is a hydrogen atom and the other is not present, and when -O-X1 is an oxo group, X4 is not present. R11 and R12 each independently denote a hydrogen atom, a hydroxy group, or an acetoxymethyl group. R13 denotes a straight-chain, branched, or cyclic, saturated hydrocarbon group having 5 to 35 carbon atoms, which may be substituted with a hydroxy group or an amino group, or a group in which ester-bonded to the ω-position of the above hydrocarbon group is a straight-chain, branched, or cyclic, saturated or unsaturated fatty acid having 8 to 22 carbon atoms, which may be substituted with a hydroxy group. R14 denotes a hydrogen atom, or a straight-chain or branched, saturated or unsaturated hydrocarbon group having 1 to 8 carbon atoms in total, which may have a substituent selected from a hydroxy group, a hydroxyalkoxy group, an alkoxy group, and an acetoxy group.

Examples of the setting polymer include polysilicone-9; a polyvinylpyrrolidone-based polymer (for example, polyvinylpyrrolidone, vinylpyrrolidone/vinyl acetate copolymer, vinylpyrrolidone/vinyl acetate/vinyl propionate ternary copolymer, vinylpyrrolidone/alkylaminoacrylate (quaternary chloride) copolymer, vinylpyrrolidone/acrylate/(meth)acrylic acid copolymer, vinylpyrrolidone/alkylaminoacrylate/vinylcaprolactam copolymer or the like); a methyl vinyl ether/maleic anhydride alkyl half ester copolymer; an acidic polyvinyl acetate-based polymer (for example, vinyl acetate/crotonic acid copolymer, vinyl acetate/crotonic acid/vinyl neodecanate copolymer, vinyl acetate/crotonic acid/vinyl propionate copolymer or the like); an acidic (meth)acrylic polymer (for example, (meth)acrylic acid/(meth)acrylic acid ester copolymer acrylic acid/alkyl acrylate ester/alkylacrylamide copolymer or the like); an amphoteric acrylic polymer (for example, N-methacryloylethyl-N,N-dimethylammonium·α-N-methylcarboxybetaine/butyl methacrylate copolymer, hydroxypropyl acrylate/butylaminoethyl methacrylate/acrylic acid octylamide copolymer or the like); an acrylamide·acrylic acid ester-based copolymer; and a chitin·chitosan compound (for example, hydroxypropylchitosan, carboxymethylchitin, carboxymethylchitosan or the like).

The hair treatment composition of the present invention has a pH at 25°C during use (during application) of 2 to 7, preferably 2.5 to 5.5, and more preferably 3 to 5, from the viewpoint of irritating properties of the composition and stability toward hydrolysis of component (a) in the composition. For adjusting this, a pH adjusting agent may be used.
With regard to the pH adjusting agent, ammonia or a salt thereof; an alkanolamine such as monoethanolamine, isopropanolamine, 2-amino-2-methylpropanol, or 2-aminobutanol, or a salt thereof; an alkanediamine such as 1,3-propanediamine or a salt thereof; a carbonate such as guanidine carbonate, sodium carbonate, potassium carbonate, sodium hydrogen carbonate, or potassium hydrogen carbonate; a hydroxide such as sodium hydroxide or potassium hydroxide, and so on may be used as an alkali agent.
Furthermore, an inorganic acid such as hydrochloric acid or phosphoric acid; a hydrochloride such as monoethanolamine hydrochloride; a phosphate such as monopotassium dihydrogen phosphate or disodium monohydrogen phosphate, and so on may be used as an acid agent.

With regard to these pH adjusting agents, an acid agent on its own, an alkali agent on its own, or a combination of the two may be used. The content thereof in the composition is preferably 0.01 to 20 mass %, and more preferably 0.1 to 15 mass %, from the viewpoint of suppression of hair damage and irritation of the scalp.

The form of the hair treatment composition of the present invention may be, for example, a liquid, an emulsion, a cream, a gel, a paste, a mousse, an aerosol or the like).

The hair treatment composition of the present invention may suitably be used in an in-bath agent such as a shampoo, a rinse, a conditioner, or a treatment or an out-bath agent such as a hair mousse, a hair mist, a hair lotion, a hair oil, or a styling agent.

A method for treating hair of the present invention is carried out by contacting the hair treatment agent explained above with hair directly or using an implement. The method of contact may employ a method that is generally and widely used for a hair treatment composition that is to be applied. Among them, in the case of a shampoo composition, there can be cited a method in which an appropriate amount thereof is contacted with the hair, massaging is carried out for a few minutes while foaming, and it is then washed away using running water. Furthermore, in the case of a rinse, a conditioner, a treatment, and so on, there can be cited a method in which an appropriate amount thereof is contacted with the hair, it is allowed to stand for a few seconds to several tens of minutes, and it is then washed away with running water.
In the case of a hair mousse, a hair oil, or a styling agent, there can be cited such as a method in which an appropriate amount thereof is contacted with the hair, and it is left as it is. The appropriate amount referred to here is an amount that gives a liquor ratio relative to the weight of hair of on the order of 1:0.005 to 1:10. The hair that is to be treated may be the whole hair or part thereof. The temperature when applied to the hair is preferably from room temperature to on the order of body temperature, but in order to promote penetration it may be heated to on the order of 50°C.

### Examples

### (Examples EA1 to EA8, Comparative Examples CA1 to CA10)

Shampoos were prepared in accordance with the formulations shown in Table 1 (Examples EA1 to EA8) and Table 2 (Comparative Examples CA1 to CA10). Hair bundles having a length of 10 cm and a width of 2 cm were washed with each of the shampoos, rinsed for 30 sec. with running water, dried with a towel, fixed via upper ends of the hair bundles, and dried naturally. A sensory test was carried out by 10 expert panelists with respect to hair softness, alignment, and luster after drying hair in accordance with the criteria below. The results are ranked by the average score of the 10 people and are shown in Table 3 and Table 4.

### <Hair softness>

- 4: Very soft.
- 3: Soft.
- 2: Could not say one way or the other.
- 1: Springy.
- 0: Very springy.

### <Hair alignment>

- 4: Alignment was very good.
- 3: Alignment was good.
- 2: Could not say one way or the other.
- 1: Alignment was poor.
- 0: Alignment was very poor.

### <Hair luster>

- 4: There was outstanding improvement in luster.
- 3: There was improvement in luster.
- 2: There was some improvement in luster.
- 1: There was no improvement in luster.
- 0: Luster disappeared.

### <Evaluation>

- AA: Average score was at least 3.5.
- BB: Average score was at least 2.5 but less than 3.5.
- CC: Average score was at least 1.5 but less than 2.5.
- DD: Average score was less than 1.5.

**[Table 1]**

| | | Example | | | | | | | |
|---|---|---|---|---|---|---|---|---|---|
| Shampoo formulation | | EA1 | EA2 | EA3 | EA4 | EA5 | EA6 | EA7 | EA8 |
| (a) | Glycylglycine | 0.5 | 0.5 | 0.5 | 0.5 | 0.5 | 0.5 | 0.5 | 0.5 |
| (b) | Malic acid | 0.5 | | | | | 0.25 | | |
| | Citric acid | | 0.5 | | | | | | |
| | Lactic acid | | | 0.5 | | 0.25 | | | 0.5 |
| | Glycolic acid | | | | 0.5 | 0.25 | 0.25 | 0.5 | |
| (b') | Toluenesulfonic acid | | | | | | | 0.5 | 0.5 |
| (d) | Laureth-2 Na sulfate (25%) | 62 | 62 | 62 | 62 | 62 | 62 | 62 | 62 |
| | Lauramide DEA | 2.3 | 2.3 | 2.3 | 2.3 | 2.3 | 2.3 | 2.3 | 2.3 |
| Others | EDTA-2Na | 0.15 | 0.15 | 0.15 | 0.15 | 0.15 | 0.15 | 0.15 | 0.15 |
| | Na chloride | 0.8 | 0.8 | 0.8 | 0.8 | 0.8 | 0.8 | 0.8 | 0.8 |
| | Na hydroxide | Appropriate amount | Appropriate amount | Appropriate amount | Appropriate amount | Appropriate amount | Appropriate amount | Appropriate amount | Appropriate amount |
| | Water | Balance | Balance | Balance | Balance | Balance | Balance | Balance | Balance |
| pH | | 3.7 | 3.7 | 3.7 | 3.7 | 3.7 | 3.7 | 3.7 | 3.7 |

**[Table 2]**

| | | Comparative Example | | | | | | | | | |
|---|---|---|---|---|---|---|---|---|---|---|---|
| Shampoo formulation | | CA1 | CA2 | CA3 | CA4 | CA5 | CA6 | CA7 | CA8 | CA9 | CA10 |
| (a) | Glycylglycine | | 0.5 | | | | | | | | |
| (b) | Malic acid | | | 0.5 | | | | | 0.25 | | |
| | Citric acid | | | | 0.5 | | | | | | |
| | Lactic acid | | | | | 0.5 | | 0.25 | | | 0.5 |
| | Glycolic acid | | | | | | 0.5 | 0.25 | 0.25 | 0.5 | |
| (b') | Toluenesulfonic acid | | | | | | | | | 0.5 | 0.5 |
| (d) | Laureth-2 Na sulfate (25%) | 62 | 62 | 62 | 62 | 62 | 62 | 62 | 62 | 62 | 62 |
| | Lauramide DEA | 2.3 | 2.3 | 2.3 | 2.3 | 2.3 | 2.3 | 2.3 | 2.3 | 2.3 | 2.3 |
| Others | EDTA-2Na | 0.15 | 0.15 | 0.15 | 0.15 | 0.15 | 0.15 | 0.15 | 0.15 | 0.15 | 0.15 |
| | Na chloride | 0.8 | 0.8 | 0.8 | 0.8 | 0.8 | 0.8 | 0.8 | 0.8 | 0.8 | 0.8 |
| | Na hydroxide | Appropriate amount | Appropriate amount | Appropriate amount | Appropriate amount | Appropriate amount | Appropriate amount | Appropriate amount | Appropriate amount | Appropriate amount | Appropriate amount |
| | Water | Balance | Balance | Balance | Balance | Balance | Balance | Balance | Balance | Balance | Balance |
| pH | | 3.7 | 3.7 | 3.7 | 3.7 | 3.7 | 3.7 | 3.7 | 3.7 | 3.7 | 3.7 |

**[Table 3]**

| | | Example | | | | | | | |
|---|---|---|---|---|---|---|---|---|---|
| Shampoo formulation | | EA1 | EA2 | EA3 | EA4 | EA5 | EA6 | EA7 | EA8 |
| Evaluation | Hair softness | AA | AA | AA | AA | AA | AA | AA | AA |
| | Hair alignment | AA | AA | AA | AA | AA | AA | AA | AA |
| | Hair luster | AA | AA | AA | AA | AA | AA | AA | AA |

**[Table 4]**

| | | Comparative Example | | | | | | | | | |
|---|---|---|---|---|---|---|---|---|---|---|---|
| Shampoo formulations | | CA1 | CA2 | CA3 | CA4 | CA5 | CA6 | CA7 | CA8 | CA9 | CA10 |
| Evaluation | Hair softness | CC | DD | DD | DD | BB | BB | BB | CC | DD | DD |
| | Hair alignment | DD | BB | BB | BB | CC | CC | CC | CC | BB | BB |
| | Hair luster | DD | BB | DD | BB | CC | CC | CC | DD | BB | BB |

As is clear from Tables 3 and 4, for a shampoo that did not contain at least one of component (a) and component (b), the score was low compared with the shampoos of the Examples with respect to improvements of all of hair softness, alignment, and luster after hair was dried.

### (Examples EB1 to EB8, Comparative Examples CB1 to CB10)

Conditioning agents were prepared in accordance with the formulations shown in Table 5 (Examples EB1 to EB8) and Table 6 (Comparative Examples CB1 to CB10). Hair bundles having a length of 10 cm and a width of 2 cm were washed with a commercial shampoo, rinsed for 30 sec. with running water, and then dried with a towel. Subsequently, they were coated with an appropriate amount of each of the conditioning agents, rinsed for 30 sec with running water, dried with a towel, fixed via upper ends of the hair bundles, and dried naturally. A sensory test was carried out by 10 expert panelists with respect to softness, alignment, and luster after drying hair in accordance with the criteria bellow. The results are ranked by the average score of the 10 people and shown in Table 7 and Table 8.

### <Hair softness>

- 4: Very soft.
- 3: Soft.
- 2: Could not say one way or the other.
- 1: Sparing.
- 0: Very springy.

### <Hair alignment>

- 4: Alignment was very good.
- 3: Alignment was good.
- 2: Could not say one way or the other.
- 1: Alignment was poor.
- 0: Alignment was very poor.

### <Hair luster>

- 4: There was outstanding improvement in luster.
- 3: There was improvement in luster.
- 2: There was some improvement in luster.
- 1: There was no improvement in luster.
- 0: Luster disappeared.

### <Evaluation>

- AA: Average score was at least 3.5.
- BB: Average score was at least 2.5 but less than 3.5.
- CC: Average score was at least 1.5 but less than 2.5.
- DD: Average score was less than 1.5.

**[Table 5]**

| | | Example | | | | | | | |
|---|---|---|---|---|---|---|---|---|---|
| Conditioner formulation | | EB1 | EB2 | EB3 | EB4 | EB5 | EB6 | EB7 | EB8 |
| (a) | Glycylglycine | 0.5 | 0.5 | 0.5 | 0.5 | 0.5 | 0.5 | 0.5 | 0.5 |
| (b) | Malic acid | 0.5 | | | | | 0.25 | | |
| | Citric acid | | 0.5 | | | | | | |
| | Lactic acid | | | 0.5 | | 0.25 | | | 0.5 |
| | Glycolic acid | | | | 0.5 | 0.25 | 0.25 | 0.5 | |
| (b') | Toluenesulfonic acid | | | | | | | 0.5 | 0.5 |
| (c) | PG | 5.25 | 5.25 | 5.25 | 5.25 | 5.25 | 5.25 | 5.25 | 5.25 |
| (d) | Steartrimonium chloride (28%) | 2.88 | 2.88 | 2.88 | 2.88 | 2.88 | 2.88 | 2.88 | 2.88 |
| | Disteardimonium chloride (75%) | 3.78 | 3.78 | 3.78 | 3.78 | 3.78 | 3.78 | 3.78 | 3.78 |
| (e) | Cetanol | 2.1 | 2.1 | 2.1 | 2.1 | 2.1 | 2.1 | 2.1 | 2.1 |
| Others | Water | Balance | Balance | Balance | Balance | Balance | Balance | Balance | Balance |
| | Na hydroxide | Appropriate amount | Appropriate amount | Appropriate amount | Appropriate amount | Appropriate amount | Appropriate amount | Appropriate amount | Appropriate amount |
| pH | | 3.7 | 3.7 | 3.7 | 3.7 | 3.7 | 3.7 | 3.7 | 3.7 |

**[Table 6]**

| | | Comparative Example | | | | | | | | | |
|---|---|---|---|---|---|---|---|---|---|---|---|
| Conditioner formulation | | CB1 | CB2 | CB3 | CB4 | CB5 | CB6 | CB7 | CB8 | CB9 | CB10 |
| (a) | Glycylglycine | | 0.5 | | | | | | | | |
| (b) | Malic acid | | | 0.5 | | | | | 0.25 | | |
| | Citric acid | | | | 0.5 | | | | | | |
| | Lactic acid | | | | | 0.5 | | 0.25 | | | 0.5 |
| | Glycolic acid | | | | | | 0.5 | 0.25 | 0.25 | 0.5 | |
| (b') | Toluenesulfonic acid | | | | | | | | | 0.5 | 0.5 |
| (c) | PG | 5.25 | 5.25 | 5.25 | 5.25 | 5.25 | 5.25 | 5.25 | 5.25 | 5.25 | 5.25 |
| (d) | Steartrimonium chloride (28%) | 2.88 | 2.88 | 2.88 | 2.88 | 2.88 | 2.88 | 2.88 | 2.88 | 2.88 | 2.88 |
| | Disteardimonium chloride (75%) | 3.78 | 3.78 | 3.78 | 3.78 | 3.78 | 3.78 | 3.78 | 3.78 | 3.78 | 3.78 |
| (e) | Cetanol | 2.1 | 2.1 | 2.1 | 2.1 | 2.1 | 2.1 | 2.1 | 2.1 | 2.1 | 2.1 |
| Others | Water | Balance | Balance | Balance | Balance | Balance | Balance | Balance | Balance | Balance | Balance |
| | Na hydroxide | Appropriate amount | Appropriate amount | Appropriate amount | Appropriate amount | Appropriate amount | Appropriate amount | Appropriate amount | Appropriate amount | Appropriate amount | Appropriate amount |
| pH | | 3.7 | 3.7 | 3.7 | 3.7 | 3.7 | 3.7 | 3.7 | 3.7 | 3.7 | 3.7 |

**[Table 7]**

| | | Example | | | | | | | |
|---|---|---|---|---|---|---|---|---|---|
| Conditioner formulation | | EB1 | EB2 | EB3 | EB4 | EB5 | EB6 | EB7 | EB8 |
| Evaluation | Hair softness | AA | AA | AA | AA | AA | AA | AA | AA |
| | Hair Alignment | AA | AA | AA | AA | AA | AA | AA | AA |
| | Hair luster | AA | AA | AA | AA | AA | AA | AA | AA |

**[Table 8]**

| | | Comparative Example | | | | | | | | | |
|---|---|---|---|---|---|---|---|---|---|---|---|
| Conditioner formulation | | CB1 | CB2 | CB3 | CB4 | CB5 | CB6 | CB7 | CB8 | CB9 | CB10 |
| Evaluation | Hair softness | CC | DD | DD | DD | BB | BB | BB | CC | DD | DD |
| | Hair Alignment | DD | BB | BB | BB | CC | CC | CC | CC | BB | BB |
| | Hair luster | DD | BB | BB | BB | CC | CC | CC | CC | BB | BB |

As is clear from Tables 7 and 8, for a conditioning agent that did not contain at least one of component (a) and component (b), in the same manner as for the above shampoos, the score was low compared with the conditioning agents of the Examples with respect to improvements of all of hair softness, alignment, and luster after hair was dried.

As hereinbefore described, in accordance with the shampoos and the conditioning agents of the Examples, which employed the hair treatment composition of the present invention, it is possible to simultaneously impart softness, luster, and alignment.

### (Examples EC1 to EC7, Comparative Examples CC1 to CC4)

Hair treatment compositions of Examples EC1 to EC7 and hair treatment compositions of Comparative Examples CC1 to CC4 shown in Table 9 were prepared. 0.1 g bundles of hair having a length of 20 cm and a width of 5 cm were made by aligning and binding the bases. The hair bundles were coated with 0.2 g of each of the hair treatment compositions, allowed to stand for 10 min, then rinsed with water at about 40°C for 30 sec, dried with a towel, fixed so that the bound part was uppermost, and dried naturally. After natural drying, the susceptibility to morning hair, ease of styling, and sustainability of styling were evaluated by the methods below.

### <Susceptibility to morning hair>

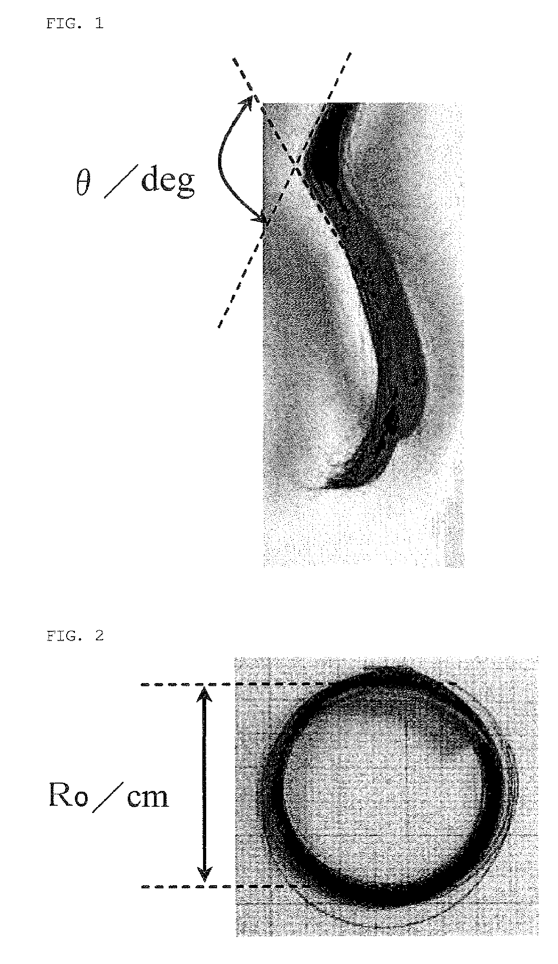

A hair bundle was placed on a towel base, which was assumed to be a pillow, in a folded-in-half state, a wig (No. 662DXLH, Beaulax Co., Ltd.) equipped with a weight so as to give an average human head weight of 6 kg was placed on the hair bundle as in a sleeping state, and it was left for 8 hours. After leaving, the hair bundle was fixed so that the bound part was uppermost, and a bending angle θ of the hair bundle when viewed side-on as shown in FIG. 1 was measured. It means that the larger the angle θ, the more the susceptibility to morning hair, and the smaller the angle θ, the more the resistance to morning hair. The results are collected in Table 9. As can be seen from Table 9 the θ of Examples EC1 to EC7 was clearly small compared with the θ of Comparative Examples CC1 to CC4, and there was resistance to morning hair.

### <Ease of styling>

The hair bundles for which susceptibility to morning hair had been evaluated were coated with 0.05 g of ion exchanged water as a mist, then wound around a rod having a diameter of 2.5 cm, and dried at 60°C. The hair bundles were removed from the rod, and with regard to curling, a curl diameter R0 of the hair bundle was measured as shown in FIG. 2, a styling rate S (%) = (2.5/R0)*100 was determined, and ease of styling was evaluated. It means that the larger the styling rate S, the more intense the curling, which means that styling is easy. The results are collected in Table 9. As can be seen from Table 9, the S of Examples EC1 to EC7 was clearly large compared with the S of Comparative Examples CC1 to CC4, and styling was easy.

### <Sustainability of styling>

A hair bundle for which ease of styling had been evaluated by the above-mentioned method was left at 20°C and 65%RH for 8 hours, and the curl diameter of the hair bundle was measured again. The curl diameter was defined after leaving. As the curl diameter R1, a styling sustain rate Δ (%) = [{S-(2.5/R1)*100}/S]*100, was determined, and styling sustainability was evaluated. It means that the smaller the styling sustain rate Δ, the higher the styling sustainability. The results are collected in Table 1. As can be seen from Table 9, it is understood that the Δ of Examples EC1 to EC7 was clearly small compared with the Δ of Comparative Examples CC1 to CC4, and the sustainability of styling was excellent.

**[Table 9]**

| Component (mass %) | | Example | | | | | | | Comparative Example | | | |
|---|---|---|---|---|---|---|---|---|---|---|---|---|
| | | EC1 | EC2 | EC3 | EC4 | EC5 | EC6 | EC7 | CC1 | CC2 | CC3 | CC4 |
| (a) | Glycylglycine | 0.2 | - | 1 | 2 | - | 0.5 | 0.5 | 0.5 | - | - | - |
| | Glycylglycylglycine | - | 0.5 | - | - | 0.5 | - | - | - | - | - | - |
| (b) | Malic acid | 2 | - | - | - | 0.25 | 0.5 | - | - | 2 | 0.5 | - |
| | Citric acid | - | 1 | - | - | - | - | - | - | - | - | - |
| | Lactic acid | - | - | 0.5 | - | - | - | 0.5 | - | - | - | - |
| | Glycolic acid | - | - | - | 0.2 | - | - | - | - | - | - | - |
| (b') | Toluenesulfonic acid | - | - | - | - | - | 0.5 | - | - | - | 0.5 | - |
| | Naphthalenesulfonic acid | - | - | - | - | - | - | 0.2 | - | - | - | - |
| Others | Na hydroxide | Appropriate amount | Appropriate amount | Appropriate amount | Appropriate amount | Appropriate amount | Appropriate amount | Appropriate amount | Appropriate amount | Appropriate amount | Appropriate amount | Appropriate amount |
| | Water | Balance | Balance | Balance | Balance | Balance | Balance | Balance | Balance | Balance | Balance | Balance |
| pH | | 3.7 | 3.7 | 3.7 | 5 | 5 | 3.7 | 3.7 | 3.7 | 3.7 | 3.7 | 3.7 |
| Susceptibility to morning hair(θ/deg) | | 16 | 18 | 14 | 13 | 16 | 12 | 11 | 40 | 35 | 34 | 47 |
| Ease of styling(S/%) | | 92 | 92 | 93 | 93 | 93 | 96 | 93 | 87 | 86 | 83 | 78 |
| Sustainability of styling(Δ/%) | | 14 | 9 | 12 | 9 | 14 | 13 | 8 | 22 | 21 | 20 | 17 |

The hair treatment composition of Example E1 and the hair treatment composition of Comparative Example E1 shown in Table 10 were prepared. Five panelists replaced the conditioner that was normally used with a hair treatment composition such that the hair treatment composition of Example ED1 was used for the left-hand side of the head and the hair treatment composition of Comparative Example CD1 was used for the right-hand side of the head for one week. Subsequently, the hair treatment composition of Comparative Example CD1 was used for the left-hand side of the head and the hair treatment composition of Example ED1 was used for the right-hand side of the head for one week. Subsequently, resistance to morning hair and ease of styling the next morning and sustainability of styling the next day were examined by questionnaire in accordance with the criteria below. The results are collected in Table 10.

### <Susceptibility to morning hair>

2 points: Example ED1 product showed more resistance to morning hair than Comparative Example CD1 product.
1 point: Example ED1 product showed slightly more resistance to morning hair than Comparative Example CD1 product.
0 points: Could not say one way or the other.
-1 points: Comparative Example CD1 product showed slightly more resistance to morning hair than Example ED1 product.
-2 points: Comparative Example CD1 product showed more resistance to morning hair than Example ED1 product.

### <Ease of styling>

2 points: Styling was easier with Example ED1 product than with Comparative Example CD1 product.
1 point: Styling was slightly easier with Example ED1 product than with Comparative Example CD1 product.
0 points: Could not say one way or the other.
-1 points: Styling was slightly easier with Comparative Example CD1 product than with Example ED1 product.
-2 points: Styling was easier with Comparative Example CD1 product than with Example ED1 product.

### <Sustainability of styling>

2 points: Styling was sustained better with Example ED1 product than with Comparative Example CD1 product.
1 point: Styling was sustained slightly better with Example ED1 product than with Comparative Example CD1 product.
0 points: Could not say one way or the other.
-1 point: Styling was sustained slightly better with Comparative Example CD1 product than with Example ED1 product.
-2 point: Styling was sustained better with Comparative Example CD1 product than with Example ED1 product.

As can be seen from Table 10, it is understood that compared with the hair treatment composition of Comparative Example CD1, the hair treatment composition of Example ED1 had better resistance to morning hair and simultaneously had ease of styling and, furthermore, the styling could be sustained.

**[Table 10]**

| Component (mass %) | | Example | Comparative Example |
|---|---|---|---|
| | | ED1 | CD1 |
| (a) | Glycylglycine | 0.5 | - |
| (b) | Malic acid | 0.5 | 0.5 |
| (c) | Propylene glycol | 5.25 | 5.25 |
| (d) | Steartrimonium chloride (28%) | 2.88 | 2.88 |
| | Disteardimonium chloride (75%) | 3.78 | 3.78 |
| (e) | Cetanol | 2.1 | 2.1 |
| Others | Na hydroxide | Appropriate amount | Appropriate amount |
| | Water | Balance | Balance |
| pH | | 3.7 | 3.7 |
| Resistance to morning hair | | 9 | |
| Ease of styling | | 8 | |
| Styling sustainability | | 9 | |

| Formulation Example EE1 (hair mist) | |
|---|---|
| | (mass %) |
| Glycylglycine | 0.1 |
| Malic acid | 4.0 |
| Steartrimonium chloride (stearyltrimethylammonium chloride) | 0.25 |
| Glycerol | 1.0 |
| Benzyl alcohol | 1.0 |
| Ethanol | 4.5 |
| PEG-10 dimethicon*1 (polyether-modified silicone) | |
| | 1.1 |
| Decylpyridinium bromide | 1.5 |
| Fragrance | 0.02 |
| Na hydroxide | adjust to pH 3.7 |
| Water | Balance |

| | |
|---|---|
| *1: KF-353A, Shin-Etsu Chemical Co., Ltd. | |

| Formulation Example EE2 (hair mist) | |
|---|---|
| | (mass %) |
| Glycylglycine | 0.2 |
| Glycolic acid | 2.5 |
| Malonic acid | 1.0 |
| 2-Benzyloxyethanol | 2.5 |
| PVP*2 (polyvinylpyrrolidone) | 3.0 |
| Ethanol | 10.0 |
| Polysilicone-9*3 | 1.0 |
| (bisisobutyl PEG-15/amodimethicon)copolymerm*4 (aminopolyether-modified silicone) | 0.5 |
| Fragrance | 0.05 |
| Na hydroxide | adjust to pH 3.7 |
| Water | Balance |

| | |
|---|---|
| *2: PVPK-30, ISP Japan, Ltd. *3: OS-88E-E, Kao Corporation *4: SS-3588, Dow Corning Toray Co., Ltd. | |

| Formulation Example EE3 (hair lotion) | |
|---|---|
| | (mass %) |
| Glycylglycine | 0.5 |
| Lactic acid | 2.0 |
| Toluenesulfonic acid | 0.5 |
| Glycerol | 2.0 |
| Benzyl alcohol | 1.0 |
| Hydroxyethyl cellulose*5 | 2.0 |
| Ethanol | 10.0 |
| Dimethicon*6 (polydimethylsiloxane) | 1.3 |
| Fragrance | 0.05 |
| Na hydroxide | adjust to pH 3.7 |
| Water | Balance |

| | |
|---|---|
| *5: HEC Daicel SE-850K, Daicel Chemical Industries, Ltd. *6: KM-9716, Shin-Etsu Chemical Co., Ltd. | |

| Formulation Example EE4 (hair lotion) | |
|---|---|
| | (mass %) |
| Glycylglycylglycine | 0.3 |
| Malic acid | 2.5 |
| Naphthalenesulfonic acid | 0.1 |
| Glycerol | 1.0 |
| Benzyl alcohol | 1.0 |
| Ethanol | 10.0 |
| Polysilicone-9*7 | 1.0 |
| Fragrance | 0.02 |
| Na hydroxide | adjust to pH 5.0 |
| Water | Balance |

| | |
|---|---|
| *7: OS-88E-E, Kao Corporation | |

| Formulation Example EE5 (hair mousse) | |
|---|---|
| | (mass %) |
| Glycylglycine | 0.1 |
| Glycylglycylglycine | 0.1 |
| Malic acid | 2.0 |
| Lactic acid | 2.0 |
| Laureth-16 | |
| (polyoxyethylene (16EO) lauryl ether) | 1.0 |
| Steartrimonium chloride (stearyltrimethylammonium chloride) | 0.1 |
| Glycerol | 1.0 |
| Benzyloxyethanol | 2.5 |
| Ethanol | 4.5 |
| Polysilicone-9*8 | 1.2 |
| Dimethicon*9 (highly polymerized methypolylsiloxane emulsion) | 0.3 |
| Dimethicon copolyol*10 (polyoxyethylene·methypolylsiloxane copolymer) | 0.2 |
| Fragrance | 0.02 |
| Na hydroxide | adjust to pH 4.0 |
| Water | Balance |

| | |
|---|---|
| *8: OS-96E-E, Kao Corporation *9: BY22-060, Dow Corning Toray Co., Ltd. *10: KF-353A, Shin-Etsu Chemical Co., Ltd. | |

| Formulation Example EE6 (shampoo) | |
|---|---|
| | (mass %) |
| Glycylglycine | 0.1 |
| Lactic acid | 0.5 |
| Malic acid | 0.5 |
| Toluenesulfonic acid | 0.3 |
| Laureth-1 ammonium sulfate (ammonium polyoxyethylene (1EO) lauryl ether sulfate) | 15.0 |
| Lauramidopropyl betaine (lauric acid amide propyl betaine) | 0.5 |
| Cocamide MEA (cocoylmonoethanolamide) | 0.3 |
| Fatty acid (C14-28)/branched fatty acid (C14-28) mixture*11 | 0.5 |
| Distearic acid PEG-250 (ethylene glycol distearate) | 3.0 |
| Isodecyl glyceryl ether | 0.5 |
| Caesalpinia spinosa hydroxypropyltrimonium chloride*12 (cationized tara gum) | 0.1 |
| Fenugreek hydroxypropyltrimonium chloride (cationized fenugreek gum) *13 | 0.1 |
| Locust bean hydroxypropyltrimonium chloride *14 (cationized locust bean gum) | 0.1 |
| Na hydroxide | adjust to pH 3.5 |
| Water | Balance |

| | |
|---|---|
| *11: CRODACID 18-MEA, Croda Japan KK *12: Catinal CTR-100, TOHO Chemical Industry Co., Ltd. *13: Catinal CF-100, TOHO Chemical Industry Co., Ltd. *14: Catinal CLB-100, TOHO Chemical Industry Co., Ltd. | |

| Formulation Example EE7 (conditioner) | |
|---|---|
| | (mass %) |
| Glycylglycine | 0.1 |
| Lactic acid | 0.7 |
| Glycolic acid | 0.7 |
| Stearoxypropyldimethylamine (N,N-dimethyl-3-octadecyloxypropylamine) | 1.0 |
| Benzyl alcohol | 0.5 |
| Isostearic acid | 0.25 |
| Shea butter | 0.25 |
| Fatty acid (C14-28)/branched fatty acid (C14-28) mixture*15 | 0.25 |
| Bismethoxypropylamido isodocosane*16 (amphiphilic amide lipid A) | 0.5 |
| Stearyl alcohol | 3.0 |
| Dimethicon*17 (polydimethylsiloxane) | 2.0 |
| Na hydroxide | adjust to pH 3.7 |
| Water | Balance |

| | |
|---|---|
| *15: CRODACID 18-MEA, Croda Japan KK *16: BRS-661C, Kao Corporation *17: SH200C, Dow Corning Toray Co., Ltd. | |

### Formulation Example EE8 (styling agent)

A stock solution was first prepared in accordance with the formulation below, and subsequently the stock solution and a propellant were charged into a pressure-resistant sealed container, thus giving an aerosol hair spray.

| 'Stock solution' | (mass %) |
|---|---|
| Glycylglycine | 0.1 |
| Lactic acid | 0.2 |
| Acrylate copolymer*18 | 5.0 |
| Ethanol | 33.5 |
| PEG-10 Dimethicon*19 (polyoxyethylene-methylpolysiloxane copolymer) | 0.2 |
| Na hydroxide | adjust to pH 6.5 |
| Water | Balance to adjust stock solution to 80% |

| | |
|---|---|
| *18: BALANCE CR, National Starch & Chemical Company *19: KF-353A, Shin-Etsu Chemical Co., Ltd. | |

| 'Propellant' | |
|---|---|
| DME (dimethyl ether) | 20.0 |

### Formulation Example EE9 (hair mousse)

A stock solution was first prepared in accordance with the formulation below, and subsequently the stock solution and a propellant were charged into a pressure-resistant sealed container, thus giving an aerosol foam.

| 'Stock solution' | (mass %) |
|---|---|
| Glycylglycine | 0.2 |
| Malic acid | 0.2 |
| Acrylate copolymer*20 | 5.0 |
| Ethanol | 33.3 |
| (C12-14) PARETH-9*21 | |
| (polyoxyethylene monoalkyl ether) | 1.0 |
| Na hydroxide | adjust to pH 6.5 |
| Water | Balance to adjust stock solution to 80% |

| | |
|---|---|
| *20: BALANCE CR, National Starch & Chemical Company *21: SOFTANOL 90, Nippon Shokubai CO., Ltd. | |

| 'Propellant' | |
|---|---|
| DME (dimethyl ether) | 20.0 |

| Formulation Example EE10 (styling agent) | |
|---|---|
| 'Stock solution' | (mass %) |
| Glycylglycine | 0.1 |
| Malic acid | 0.1 |
| Toluenesulfonic acid | 0.2 |
| Cetearyl alcohol (cetostearyl alcohol) | 4.0 |
| Paraffin | 16.5 |
| Vaseline | 10.0 |
| Mineral oil (liquid paraffin) | 3.0 |
| Ceteth-20 (polyoxyethylene cetyl ether (20 E.O.)) | 5.0 |
| Glyceryl stearate (glycerol monostearate) | 3.0 |
| Laureth-4 Na phosphate (sodium polyoxyethylene lauryl ether phosphate) | 0.5 |
| Propylparaben (propyl p-oxybenzoate) | 0.1 |
| Octyldodecyl myristate | 3.0 |
| Carbomer*22 (carboxyvinyl polymer) | 0.1 |
| PEG-32 (polyethylene glycol 1540) | 5.0 |
| Na stearoyl methyl taurate (sodium N-stearoyl-N-methyltaurate) | 1.0 |
| Methylparaben (methyl p-oxybenzoate) | 0.2 |
| BG (butylene glycol) | 5.0 |
| EDTA-2Na (disodium edetate) | 0.1 |
| PVP*23 (polyvinylpyrrolidone) | 0.8 |
| Na benzoate | 0.2 |
| Phenoxyethanol | 0.2 |
| Na hydroxide | adjust to pH 6.5 |
| Water | Balance |

| | |
|---|---|
| *22: Carbopol 980, Lubrizol Advanced Materials, Inc. *23: PVP K-30, ISP Japan, Ltd. | |

## Claims

1. A hair treatment composition, comprising components (a) and (b) :
(a) a compound having 2 or 3 amino acid residues and represented by general Formula (1), or a salt thereof 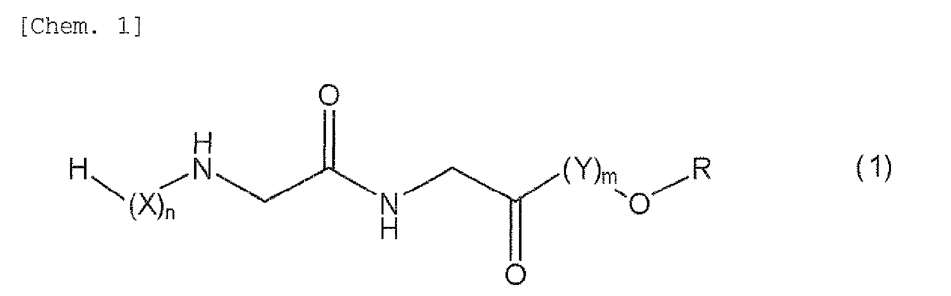 [in the formula, X denotes a divalent hydrocarbon group having 1 to 4 carbon atoms, which may be substituted with a hydroxy group, or an amino acid residue selected from an arginine residue, an alanine residue, a phenylalanine residue, a glycine residue, a glutamine residue, a glutamic acid residue, a serine residue, a proline residue, an N-methylproline residue, and a 4-hydroxyproline residue,
Y denotes an amino acid residue selected from an arginine residue, an alanine residue, a glycine residue, a glutamine residue, a glutamic acid residue, a serine residue, a proline residue, and a 4-hydroxyproline residue or a divalent group represented by chemical Formula (2) 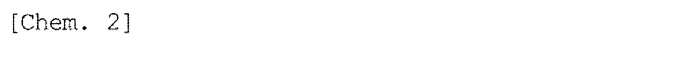 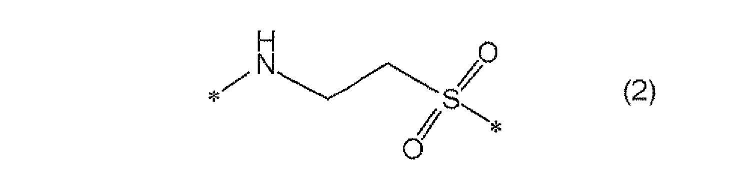 (in the formula, -* denotes a direct bond bonding to an adjacent carbonyl group or oxygen atom),
R denotes a hydrogen atom or a monovalent hydrocarbon group having 1 to 4 carbon atoms, which may be substituted with a hydroxy group, m and n denote 0 or 1, and when m and n are simultaneously 1, X is not an amino acid residue]; and
(b) an aliphatic carboxylic acid having no greater than 8 carbon atoms or a salt thereof.

2. The composition according to claim 1, further comprising (b') an aromatic sulfonic acid having no greater than 10 carbon atoms or a salt thereof.

3. The composition according to claim 1 or 2, wherein the compound as component (a) is glycylglycine or glycylglycylglycine.

4. The composition according to any one of claims 1 to 3, wherein the aliphatic carboxylic acid having no greater than 8 carbon atoms or a salt thereof as component (b) is malic acid, citric acid, lactic acid, glycolic acid, glyceric acid, or tartaric acid.

5. The composition according to any one of claims 1 to 4, wherein the aromatic sulfonic acid having no greater than 10 carbon atoms or a salt thereof as component (b') is p-toluenesulfonic acid or naphthalenesulfonic acid.

6. The composition according to any one of claims 1 to 5, wherein it has a pH at 25°C of 2 to 7.

7. A method for treating hair using the hair treatment composition according to any one of Claims 1 to 6.
